# EUROPEAN PATENT SPECIFICATION

(11) **EP 4 063 855 B1**
(45) Date of publication and mention of the grant of the patent: **17.12.2025**
(21) Application number: 20890489.6
(22) Date of filing: 14.10.2020
(51) Int. Cl.: G01N 33/49, A61B 5/145, G01N 1/40

(54) **MULTI-FILTER FOR SEPARATING BLOOD COMPONENTS, AND DISEASE DIAGNOSIS KIT USING SAME**
MULTIFILTER ZUR TRENNUNG VON BLUTKOMPONENTEN UND KRANKHEITSDIAGNOSEKIT DAMIT
FILTRE MULTIPLE PERMETTANT LA SÉPARATION DE COMPOSANTS SANGUINS, ET KIT DE DIAGNOSTIC DE MALADIE UTILISANT CE DERNIER

(30) Priority: 19.11.2019 KR 20190148466
(43) Date of publication of application: 28.09.2022
(73) Proprietor: Kyungpook National University Industry-Academic Cooperation Foundation, Daegu 41566 (KR); Kyungpook National University Hospital, Jung-gu Daegu 41944 (KR)
(72) Inventor: HAN, Hyungsoo, Daegu 41186 (KR); LEE, Youngmi, Daegu 41415 (KR); BAE, Mijung, Daegu 41521 (KR)
(74) Representative: Weickmann & Weickmann PartmbB
(86) International application number: PCT/KR2020/013957
(87) International publication number: WO 2021/101068

(56) References cited:
- JP-A- 2015 500 464
- JP-B2- 3 242 993
- JP-B2- 3 242 993
- KR-B1- 101 463 560
- US-A1- 2013 184 446
- US-A1- 2018 126 383
- US-A1- 2019 049 353
- US-A9- 2016 349 156

## Description

### [Technical Field]

This disclosure relates to a multi-layered filter for separating blood components and a disease diagnosis kit using the same, and more specifically, to a multi-layered filter for separating blood components, which includes a first filter unit for separating blood cells from a whole blood sample, a second filter unit for separating cell fragments having blood platelet and exosome, and a third filter unit for separating proteins, wherein the whole blood sample is separated into blood components through each filter unit provided to have various pore sizes, and after filtering, the multi-layered filter may be disassembled such that each component remaining in the filters is collected and used for diagnosis, and a disease diagnosis kit using the same.

### [Background Art]

Blood includes cells such as white blood cells and red blood cells, small particles derived from cells such as blood platelets and exosomes, and trace molecules including plasma proteins, lipids, nucleic acid fragments and metabolites.

**If** individual components of the blood are measured, health status and disease may be diagnosed, so the blood is being used as a useful biological sample for diagnosis.

Since it is difficult to use blood directly in the measurement method used for diagnosis, a technique for measuring purely separated individual components is applied after a treatment procedure is performed to separate blood components.

However, the methods currently used to separate individual components from blood require complex procedures, and various instruments and reagents are required to perform each procedure, so there is a disadvantage that the methods can be performed only in well-equipped laboratories.

Patent Literature 1 discloses a plasma separation chip having a radial filter structure, which includes an upper substrate and a lower substrate and has a blood inlet and a plasma outlet, wherein one of the substrates has a plasma transfer channel for moving plasma using capillary force and includes a first filter unit for filtering leukocytes and a second filter unit for filtering red blood cells in order along the movement path of plasma, and it reports that the plasma separation chip rapidly and efficiently removes plasma from blood without a separate driving means. However, this disclosure is limited to separating only plasma from blood.

In addition, Patent Literature 2 relates to a leukocyte removal filter device and a leukocyte removal method for removing microaggregates or leukocytes that cause side effects of transfusion from whole blood preparations for transfusion, red blood cell preparations, blood platelet preparations, plasma preparations, and the like, and it proposes a leukocyte removal filter device and a leukocyte removal method that exhibit excellent filtration performance under both use conditions of room temperature filtration and refrigerated filtration. However, this disclosure is also directed to a method for separating a specific component from blood and is limited to laboratory practice, with poor field applicability.

Furthermore, US2016349156 discloses a filter assembly for blood analysis with three sequential filter units which may be disassembled for analysis.

Accordingly, inventors of this application have made efforts to simplify conventional complex procedures such as the use of various devices and reagents for separating individual components of blood, and, by using the point that individual components of the blood have different sizes and physicochemical properties, the inventors have found that individual components of blood may be efficiently separated without a separate machine by providing a multi-layered filter so that the whole blood sample is separated into blood components through various filter units with various pore sizes, and after filtering, the multi-layered filter may be disassembled such that each component remaining in the filters is collected and used for diagnosis, thereby having completed the present disclosure.

### [Prior Literature]

### [Patent Literature]

Korean Patent No. 1190035 (published on October 12, 2012)
Korean Patent No. 0876819 (published on January 7, 2009)

### [Disclosure]

### [Technical Problem]

This disclosure is directed to providing a multi-layered filter that may separate blood components from whole blood.

### [Technical Solution]

The present disclosure provides a multi-layered filter according to claim 1. Further advantageous modifications are laid out in the dependent claims.

### [Advantageous Effects]

The multi-layered filter for separating blood components according to the present disclosure may conveniently extract and separate individual components of blood on the spot without the help of special equipment and use the individual components for diagnosis.

In addition, even though various instruments and reagents are generally used to separate individual components of blood, in the present disclosure, it is possible to effectively separate individual components of blood by using a multi-layered filter without a separate machine.

The multi-layered filter for separating blood components according to the present disclosure simplifies the procedure for separating individual components from blood, so blood tests may be performed even in medical sites that are not equipped with many equipment or facilities, thereby securing field applicability.

Further, in the multi-layered filter of the present disclosure, the filter units are combined in order, and after filtering, the combined filter units are disassembled to diagnose each component, so it may be applied as a disease diagnosis kit.

### [Description of Drawings]

FIG. 1 is a sectional view showing a multi-layered filter for separating blood components according to the first embodiment of the present disclosure,
FIG. 2 is a sectional view showing a multi-layered filter for separating blood components according to the second embodiment of the present disclosure,
FIG. 3 shows a separation experiment result of blood components using the multi-layered filter according to Example 1 of the present disclosure,
FIG. 4 shows a separation experiment result of blood components using the multi-layered filter according to Example 2 of the present disclosure,
FIG. 5 shows a result of experimenting whether plasma components remain in a third filter unit, in the separation experiment result of blood components using the multi-layered filter according to Example 2 of the present disclosure,
FIG. 6 shows a result of experimenting whether plasma components remain according to a pore size of the third filter unit, in the separation experiment result of blood components using the multi-layered filter according to Example 2 of the present disclosure,
FIG. 7 shows a result of experimenting whether nucleic acid remains in a fourth filter unit, in the separation experiment result of blood components using the multi-layered filter according to Example 2 of the present disclosure,
FIG. 8 shows a multi-layered filter prepared according to Comparative Example 1 of the present disclosure and a blood separation result using the same,
FIG. 9 shows a multi-layered filter prepared according to Comparative Example 2 of the present disclosure and a blood separation result using the same, and
FIG. 10 shows a multi-layered filter prepared according to Comparative Example 3 of the present disclosure and a blood separation result using the same.

### [Best Mode]

Hereinafter, the present disclosure will be described in detail.

In the present disclosure, by using the fact that all components contained in whole blood have different sizes and physicochemical properties, a multi-layered filter according to the present disclosure is configured by combining a plurality of filter units with different pore sizes and materials suitable for each component to be separated and obtained, so that different components may remain in or pass through each filter unit while passing through the filter units using capillary action.

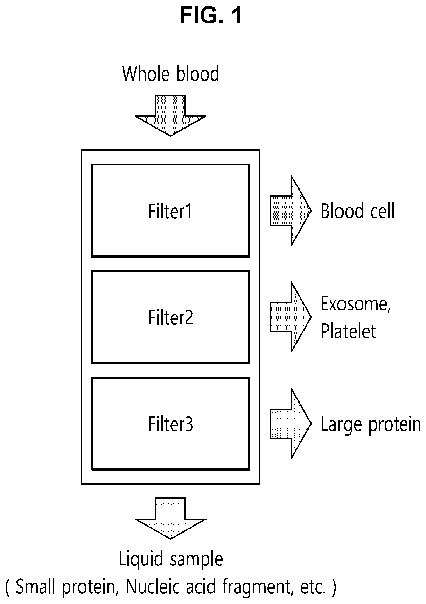

FIG. 1 is an example of a sectional view of a multi-layered filter for separating blood components according to the first embodiment of the present disclosure, and the multi-layered filter is designed to have different physical properties such as pore sizes and pore structures, which does not allow a component to pass in each stage, so that the corresponding component may be separated from a whole blood sample.

Specifically, the present disclosure provides a multi-layered filter, which includes a first filter unit for separating blood cells from a whole blood sample, a second filter unit for separating cell fragments having blood platelets and exosomes, and a third filter unit for separating proteins, wherein the whole blood sample is separated into blood components by passing through the filter units with different pore sizes.

In addition, in the multi-layered filter, the third filter unit may further include an additional filter unit according to a size of the protein to be separated.

In addition, small protein or nucleic acid component passing through the third filter unit may be separated using a separate filter unit (a fourth filter unit).

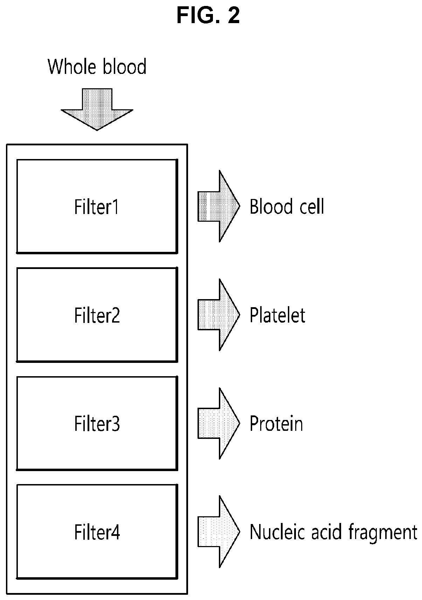

FIG. 2 is a sectional view showing a multi-layered filter for separating blood components according to the second embodiment of the present disclosure, and a fourth filter unit for separating a small protein or nucleic acid component passing through the third filter unit may be further included.

In addition, in the multi-layered filter of the present disclosure, the filter units may be combined in order, and after filtering, the multi-layered filter may be disassembled so that each component remaining in each filter unit may be used as a diagnostic or experimental material.

### 1. First filter unit (Filter1)

The first filter unit (Filter1) is used for separating blood cells from the administered whole blood sample, and it must have a pore size and structure that does not allow blood cells to pass therethrough and simultaneously must be made of a material that is not adsorbed to other components such that other components may easily pass therethrough. Specifically, the material may be selected from the group of natural fibers, and it is preferable as long as the material allows the filter to have a large pore size.

In addition, in order to separate blood cells without damage, the filter must satisfy standard requirements, namely a pore size of 5-15 µm, thickness of 500-1500 µm, weight of 100-400 gram/m², micron rating of 2-5 µm, and hole-up-volume of 20-100 µL.

The most preferred filter standards employed in the embodiment of the present disclosure are pore size of 10 µm, thickness of 1000 µm, weight of 250 gram/m², micron rating of 3 µm, and hole-up-volume of 50 µL.

In the multi-layered filter of the present disclosure, blood cells remain in the first filter unit, and the blood cells separated through the first filter unit and remaining therein may be used as an experimental sample.

### 2. Second filter unit (Filter2)

The second filter unit (Filter2) is used for separating particles other than the blood cells according to their size.

That is, the second filter unit used for separating fragments smaller than cells, such as blood platelet, cell fragments and exosomes, must have a pore size and structure that does not allow the cell fragments to pass, and at the same time, the second filter unit must be made of a material that allows other components such as plasma protein to pass through the filter easily and is not adsorbed to the filter.

The filter has a pore size of 0.05-1 µm, thickness of 100-1000 µm, weight 100-300 gram/m², micron rating of 1-5 µm, and hole-up-volume of 2-10 µL, and more preferably, it satisfies the filter standards of pore size of 0.1 µm, thickness of 300 µm, weight of 200 gram/m², micron rating of 2 µm, and hole-up-volume of 5 µL.

Through the second filter unit (Filter2), the cell fragments remain, and the blood platelet, cell fragments, exosomes, etc. separated through the second filter unit (Filter2) may be used as experimental samples.

### 3. Third filter unit (Filter3)

The third filter unit (Filter3) is a filter used for separating proteins and must have a pore size and structure that does not allow protein to pass, and at the same time use a material with very low affinity and adsorption for nucleic acids or proteins.

In addition to the second filter unit (Filter2) and the third filter unit (Filter3) presented in FIG. 1, a filter capable of more finely separating small-sized and medium-sized proteins or other particles present in blood by adjusting filter standards (pore size, structure, number of filters, etc.) according to the size or characteristics desired by the experimenter may be further added.

Therefore, by using two or more filters, the third filter unit (Filter3) may allow various particle components of various sizes such as proteins present in blood to remain in each filter unit so that the components are separated into each other.

The protein components remain through the third filter unit (Filter3), and the remaining protein separated through the third filter unit (Filter3) may be used as an experimental sample.

### 4. Fourth filter unit (Filter4)

If it is necessary to separate only nucleic acid fragments from the liquid sample that has passed through the third filter unit (Filter3), a fourth filter unit (Filter4) may be added.

The fourth filter unit (Filter4) may use a material to which nucleic acids are easily adsorbed. Nucleic acid remains in the fourth filter unit (Filter4), and the remaining nucleic acid components may be used as experimental samples.

In addition, in the liquid sample that has passed through the third filter unit (Filter3), very small-sized particles such as amino acids, bases and various metabolites or components dissolved in liquids exist, so they may be used for diagnostic tests.

At this time, the sample may be used directly as it is in the liquid state that has passed through the third filter unit (Filter3), or may be used by remaining in the fourth filter unit (Filter4) for the purpose of preservation or transportation. If necessary, the sample may be used by adding other sample preservation methods such as drying and freezing in a state of remaining in the fourth filter unit (Filter4).

A preferred material of the fourth filter unit (Filter4) is a silica material to help adsorption of nucleic acids, and a commercially available filter material made of silica for separating nucleic acids may be used.

The multi-layered filter of the present disclosure as described above is designed such that the filter units are combined very close to each other and are spaced apart from each other to form a space between the filter units.

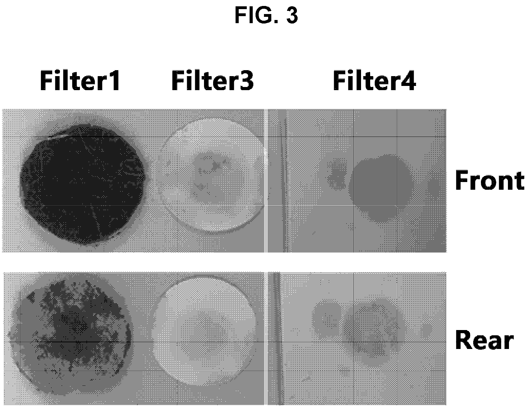

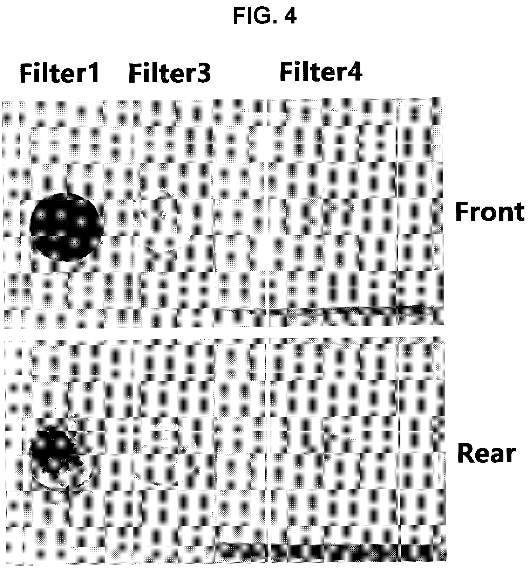

FIG. 3 shows a blood separation result using a multi-layered filter prepared according to Example 1 of the present disclosure, and FIG. 4 shows a blood separation result using a multi-layered filter prepared according to Example 2 of the present disclosure. Here, in the multi-layered filter configured such that the filter units are combined very close to each other and are spaced apart from each other to form a space between the filter units, it may be found that blood cells are separated and absorbed in the first filter unit, the other components pass through the third filter unit, and only pure plasma components pass through the fourth filter unit to be separated.

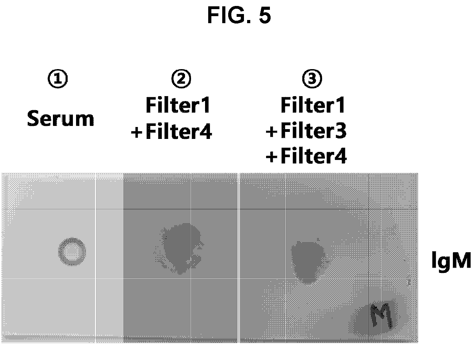

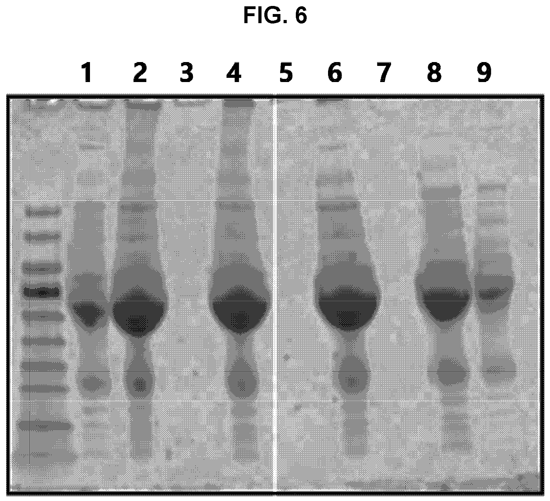

FIGS. 5 and 6 show a result of experimenting whether plasma components remain in the third filter unit, in the separation experiment results of blood components using the multi-layered filter. Here, it may be found that proteins may be selectively separated. As a result of checking whether plasma components are separated according to the pore size of the third filter unit (Filter3), it may be found that the proteins do not pass through the third filter unit (Filter3) in the case of pore sizes of 0.003 µm, 0.005 µm and 0.01 µm and almost remain in the third filter unit (Filter3).

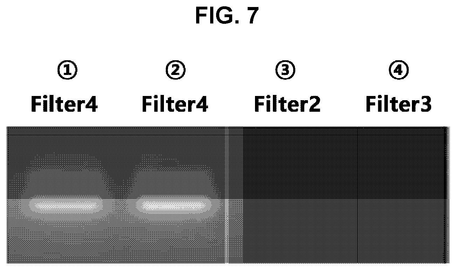

FIG. 7 shows a result of experimenting whether nucleic acid remains in the fourth filter unit, in the separation experiment result of blood components using the multi-layered filter of the present disclosure. Here, when the PCR is found with a human GAPDH primer, a band was not observed in the second filter unit (Filter2) and the third filter unit (Filter3), but a band was observed only in the fourth filter unit (Filter4), thereby finding that the nucleic acid remains.

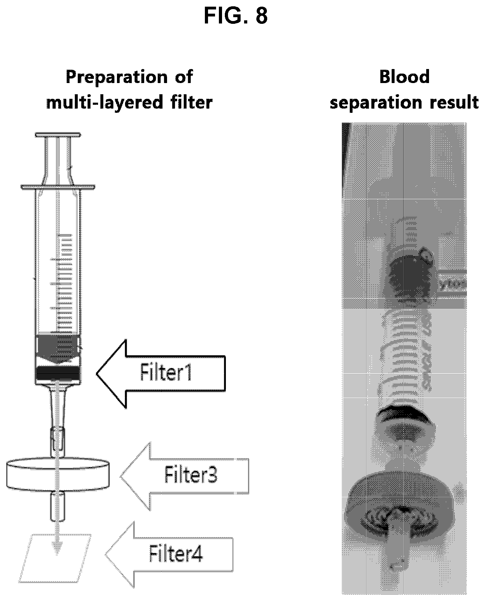

Meanwhile, FIGS. 8 to 10 show multi-layered filters prepared according to Comparative Examples 1 to 3 and blood separation results using the same. Here, if the multi-layered filter is prepared by compressing filter units with a pressure using a dice by means of O-rings as in the prior art, the blood is intensively moved to a specific area not to secure proper separation. That is, blood cells are not completely separated in the first filter unit, but partially moved to the third filter unit and the fourth filter unit.

From the above, in the structure of the multi-layered filter of the present disclosure, the filter units should be as closer to each other as possible, but there should be no physical interference, such as external pressure, that damages the basic structure of the filters.

In addition, in using the multi-layered filter of the present disclosure, damage to blood cells should be minimized when the whole blood is injected into the multi-layered filter.

At this time, damage to blood cells causes contamination of plasma components by cell components, leading to incorrect analysis results and hindering the separation of components of the whole blood.

20-100 µL of whole blood sample may be injected into the multi-layered filter of the present disclosure to separate blood components, so it is possible to collect blood from a finger or the like using a blood glucose meter and immediately use the blood at home as well as in a hospital or laboratory.

Therefore, the multi-layered filter of the present disclosure may simply extract and separate individual components without the help of special equipment so that the individual components are used for diagnosis.

Furthermore, the present disclosure may provide a disease diagnosis kit using the multi-layered filter described above.

As a preferred example, the present disclosure provides a cerebral stroke diagnosis kit capable of measuring blood P-selectin using the multi-layered filter described above.

P-selectin is a protein expressed on the surface of activated endothelial cells and is activated in blood platelets to express its functions. Under normal circumstances, the P-selectin is located on the surface of endothelial cells and blood platelet, but when exposed to diseases such as stroke, it is converted to sP-selectin (soluble platelet selectin).

That is, sP-selectin changed to a soluble form by disease is leaked into the plasma. Therefore, measuring sP-selectin leaked into plasma may be utilized for disease diagnosis.

If the whole blood passes through the multi-layered filter of the present disclosure, blood cells are separated in the first filter unit (Filter1) and the blood platelets are separated in the second filter unit (Filter2-1), so it is possible to check P-selectin in an inactive state.

In the second filter unit (Filter2-2), which has a smaller pore size than the second filter unit (Filter2-1), proteins in plasma may be separated. Here, if proteins are separated from the blood of stroke patients, activated forms such as sP-selectin may be found.

In another case, P-selectin is sometimes elevated due to an inflammatory response, but it may also be elevated in relation to stroke. In order to distinguish two cases from each other, the gene and protein expression levels may be checked together to make a clearer diagnosis. That is, in case of inflammation, the gene expression of inflammation-related protein increases together with P-selectin, but in case of stroke, only P-selectin protein increases.

Hereinafter, the present disclosure will be described in more detail through examples.

The following examples are intended to describe the present disclosure in more detail, and the scope of the present disclosure is not limited to these examples.

### <Example 1> Preparation of a multi-layered filter

A multi-layered filter was prepared by stacking the fourth filter unit, the third filter unit, the second filter unit and the first filter unit on an acrylic plate in order. The filter units were combined very close to each other, but they were fixed with a little space being formed therebetween.

The first filter unit was prepared with filter standards of pore size of 10 µm, thickness 1000 of µm, weight of 250 gram/m², micron rating of 3 µm, and hole-up-volume of 50 µL, and was made of natural fiber to separate blood cells without damage. In addition, the second filter unit was prepared with filter standards of pore size of 0.1 µm, thickness of 300 µm, weight of 200 gram/m2, micron rating of 2 µm, and hole-up-volume of 5 µL. Each filter unit is round with diameter of 13 mm, and 100 µL of whole blood was absorbed onto the first filter unit, and after 2 minutes, it was checked whether the blood passed by using an end of the capillary or tip, and the filter units were disassembled to check front and rear sides thereof.

As shown in FIG. 3, it was found that blood cells were separated in the first filter unit (Filter1) and then blood cells did not pass through the third filter unit (Filter3) and the fourth filter unit (Filter4), and plasma was completely separated.

### <Example 2> Preparation of a multi-layered filter

A multi-layered filter was prepared in the same manner as in Example 1, except that the amount of whole blood used in Example 1 was changed to 20 µL. At this time, according to the change of blood volume, the size of all filters used in the multi-layered filter was reduced to 4 mm.

The administration amount of the whole blood was determined by the amount obtained when an individual collects blood from a finger or the like with a collection needle using a blood glucose meter at home, rather than using a syringe at a hospital to collect blood from blood vessels.

As a result, as shown in FIG. 4, blood cells were separated and absorbed in the first filter unit (Filter1), the other components passed through the third filter unit (Filter3), and only the pure plasma component passed through the fourth filter unit (Filter4) to be separated, so it was confirmed that filter change is also possible.

### <Comparative Example 1> Preparation of a multi-layered filter by applying pressure

The first filter unit (Filter1) prepared in a circular shape with a diameter of 13 mm was inserted into a 1 mL syringe, the third filter unit (Filter3) was coupled thereto in the form of a syringe filter, the fourth filter unit (Filter4) was connected to a lower position thereof, and 100 µL of whole blood was injected therein so that the whole blood is separated by passing the filter while applying pressure using the piston of the syringe.

As a result, as shown in FIG. 8, after the whole blood passed through all of the multi-layered filter units, a red color was found as a whole. Therefore, when the pressure was applied, red blood cells were broken, and blood cells were not properly separated in the first filter unit. Therefore, although it is possible to shorten the time when applying pressure, since blood components cannot be completely separated due to destruction of blood cells or the like, a method of applying pressure is excluded when preparing a multi-layered filter.

### <Comparative Example 2> Preparation of a multi-layered filter without pressure

A multi-layered filter was prepared by coupling two acrylic plates and two O-rings and installing a multi-layered filter between the O-rings to be very close to each other. The multi-layered filter was designed so that liquid moves by capillary force even in the absence of pressure. In the multi-layered filter, the first filter unit (Filter1), the third filter unit (Filter3) and the fourth filter unit (Filter4) manufactured in a circular shape with a diameter of 13 mm were stacked in order, and at this time, 100 µL of whole blood was moved by capillary force through the sample injection hole. After 2 minutes passed from injection of 100 µL of blood, the multi-layered filter was disassembled to check the status of each filter unit.

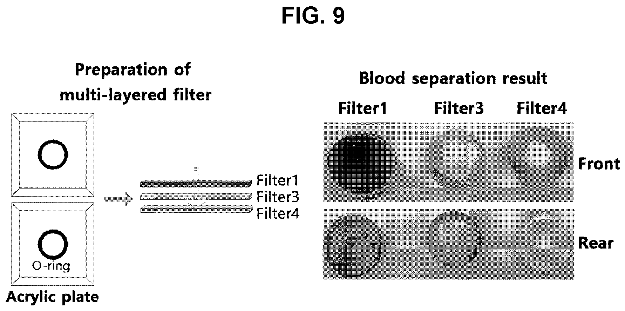

As a result, as shown in FIG. 9, although direct pressure was not applied while blood was passing, it was found that blood cells were still not completely separated in the first filter unit (Filter1) but partially moved to the third filter unit (Filter3) and the fourth filter unit (Filter4).

### <Comparative Example 3> Preparation of a multi-layered filter

In the case where the filter itself has the same capacity in the multi-layered filter prepared according to the Comparative Example 2, the blood separation experiment of the multi-layered filter was performed by adjusting the spatial volume generated by the O-ring and the administered whole blood volume.

Specifically, the multi-layered filter was manufactured in a circular shape with a diameter of 13 mm, the inner diameter of the O-ring was fixed to 10 mm smaller than that, and the thickness was changed to 1.5 mm, 1 mm and 0.6 mm. At this time, the administration volume of whole blood was 20 µL, which is the amount of blood when blood was collected from a finger or the like with a collection needle. After 2 minutes from the administration, the filter units were disassembled to check front and back sides thereof.

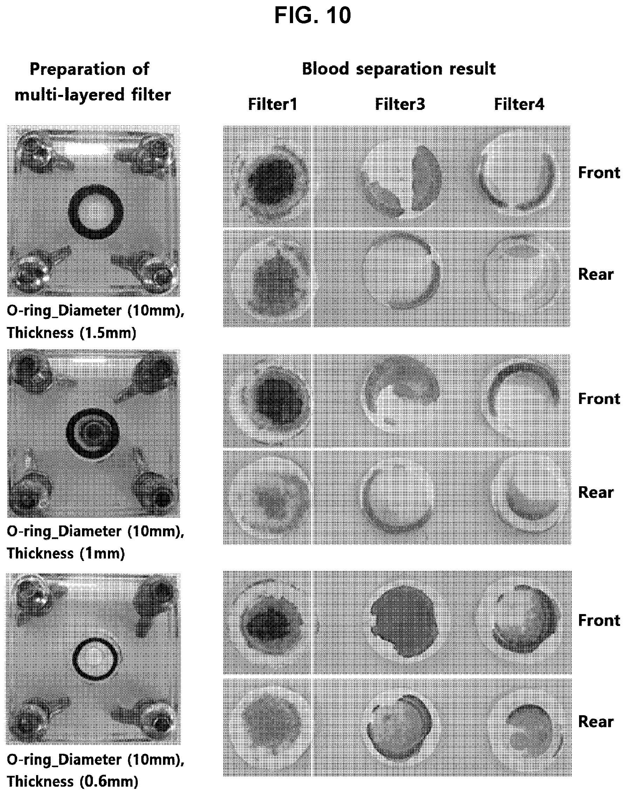

As the results are shown in FIG. 10, blood cells were still not separated properly through the first filter unit (Filter1). However, despite that blood was injected at the center of the inlet, in the third filter unit (Filter3) and the fourth filter unit (Filter4), blood was observed at a position in contact with the O-ring.

Therefore, it is expected that the blood is absorbed into the junction portion of the filter and the O-ring by the pressure of a region where the O-rings positioned at the front and rear sides of the acryl plate are engaged with each other, so that the blood cells cannot be separated and but pass therethrough as they are. Accordingly, the result caused by the structural deformation of the filter unit due to the pressure between the filter unit and the O-ring was checked.

### <Experimental Example 1> Separation performance evaluation 1

In the experimental results using the multi-layered filter prepared according to the Example 2, it was found that blood cells were reliably separated in the first filter unit (Filter1).

In the next step, in the liquid sample remaining in the fourth filter unit (Filter4), in order to determine whether plasma proteins are present, plasma proteins were checked by western blot or dot blot method. After the antigen-antibody reaction was completed, a color change was induced using 3,3',5,5'-tetramethylbenzidine (TMB) dye, which developed blue, and observed with the naked eye.

An antibody capable of detecting IgM, which is the largest type of antibody among plasma proteins, was used to react with the blood components remaining in the fourth filter unit (Filter4) and was checked by blot analysis. To test the filter performance, three samples were used to be compared, and there were used ① serum separated from blood by centrifugation, ② components that passed directly through the first filter unit (Filter1), and ③ components that passed the first filter unit (Filter1) and the third filter unit (Filter3) and separated in the fourth filter unit (Filter4).

As a result, as shown in FIG. 5, it was found that IgM appeared in all of three experimental conditions. Therefore, it was found that the selective separation of proteins is possible with the multi-layered filter of the present disclosure.

### <Experimental Example 2> Separation performance evaluation 2

In the experimental results using the multi-layered filter prepared according to the Example 2, an experiment was performed to check the change of proteins capable of passing according to the pore size of the third filter unit (Filter3) when blood passed through the multi-layered filter.

The pore size of the third filter unit (Filter3) used for the experiment was selected as 003 µm, 0.005 µm, 0.01 µm and 0.03 µm. In order to identify proteins remaining in the fourth filter unit (Filter4) and each protein not passing therethrough after passing through the third filter unit (Filter3), electrophoresis on SDS-Page gel was used, and Coomassie blue staining method was used.

An electrophoresis experiment was performed to compare and check whether proteins in the blood passed through the third filter unit (Filter3) or did not pass therethrough but remained.
No. 1: Protein that passed only the first filter unit (Filter1)
No. 2: Protein remaining after not passing through the third filter unit (Filter3) of 0.003 µm
No. 3: Protein that passed through the third filter unit (Filter3) of 0.003 µm and remained in the fourth filter unit (Filter4)
No. 4: Protein that did not pass through the third filter unit (Filter3) of 0.005 µm and remained
No. 5: The protein that passed through the third filter unit (Filter3) of 0.005 µm and remained in the fourth filter unit (Filter4)
No. 6: Protein that did not pass through the third filter unit (Filter3) of 0.01 µm and remained
No. 7: Protein that passed through the third filter unit (Filter3) of 0.01 µm and remained in the fourth filter unit (Filter4)
No. 8: Protein that did not pass through the third filter unit (Filter3) of 0.03 µm and remained
No. 9: Protein that passed through the third filter unit (Filter3) of 0.03 µm and remained in the fourth filter unit (Filter4)

As a result, as shown in FIG. 6, it was found that the blood proteins did not pass through the third filter unit (Filter3) with the pore sizes of 0.003 µm, 0.005 µm and 0.01 µm and most of them remained in the third filter unit (Filter3).

Meanwhile, if the pore size of the third filter unit (Filter3) was 0.03 µm, it was found that a part of the proteins passed therethrough to the fourth filter unit (Filter4). Therefore, in the case of using the third filter unit (Filter3) for separation, it is possible to control the separation range according to the size by adjusting the pore size to around 0.03 µm.

### <Experimental Example 3> Separation performance evaluation 3

From the experimental results using the multi-layered filter prepared according to the Example 2, it was found that the first filter unit reliably separated blood cells. Then, in order to check whether the nucleic acid passed through the first filter unit (Filter1) and the third filter unit (Filter3) and remained in the fourth filter unit (Filter4), DNA was extracted from the nucleic acid remaining in the fourth filter unit (Filter4) and PCR was performed with human GAPDH primer to confirm the expression of the gene.

In FIG. 7, ① and ② show the results of the fourth filter unit (Filter4), ③ shows the gene expression through PCR by extracting each gene from the second filter unit (Filter2), and ④ shows the gene expression through PCR by extracting each gene from the third filter unit (Filter3).

As a result, in the fourth filter unit (Filter4), when PCR was checked with the human GAPDH primer, it was found that the nucleic acid passed through and separated by checking the band. Also, in the second filter unit (Filter2) and third filter unit (Filter3), since the band was out checked, it was found that all nucleic acids passed therethrough and did not remain.

In the above, the present disclosure has been described in detail only with respect to the embodiments, but it is obvious to those skilled in the art that various changes and modifications can be made within the scope of the appended claims.

## Claims

1. A multi-layered filter for separation of blood components immediately at a medical spot comprising:
a first filter unit configured to separate blood cells from a whole blood sample;
a second filter unit configured to separate cell fragments including blood platelet or exosome; and a third filter unit configured to separate protein,
and wherein the filters of the first, second and third units have different pore sizes and are sequentially combined by decreasing pore size and are spaced apart from each other with a space therebetween and wherein the combined filter units emites are configured to be disassembled for diagnosis of each blood component, and wherein the whole blood sample is separated by passing through the filter units,
**characterized in that** the pore size of the first filter unit is in a range of 5 µm to 15 µm, and the pore size of the second filter unit is in a range of 0.05 µm to 1 µm, and the pore size of the third filter unit is in a range of 0.003 µm to 0.01 µm.

2. The multi-layered filter according to claim 1, further comprising:
a fourth filter unit configured to separate small protein or nucleic acid components passed through the third filter unit and wherein the fourth filter unit is preferably made of silica material for improved nucleic acids adsorption.

3. The multi-layered filter according to any of claims 1 - 2, wherein the multi- layered filter is configured to separate the whole blood sample administrated in an amount of 20 - 100 µL.

4. The multi-layered filter according to any of claims 1 - 3, wherein the first filter unit has a thickness of 500 - 1500 µm, a weight of 100 - 400 gram/m², a micron rating of 2 - 5 µm, and a hole up-volume of 20 - 100 µL.

5. The multi-layered filter according to any of claim 4, wherein the pore size is 10 µm, the thickness is 1000 µm, the weight is 250 gram/m², the micron rating is 3 µm, and the hole-up-volume is 50 µL.

6. The multi-layered filter according to any of claims 1 - 5, wherein the second filter unit has a thickness of 100 - 1000 µm, a weight of 100 - 300 gram/m², a micron rating of 1 - 5 µm, and a hole-up-volume of 2 - 10 µL.

7. The multi-layered filter according to claim 6, wherein the pore size is 0.1 µm, the thickness is 300 µm, the weight is 200 gram/m², the micron rating is 2 µm, and the hole-up-volume is 5 µL.

## Patentansprüche

1. Mehrschichtiger Filter zur Trennung von Blutbestandteilen unmittelbar an einem medizinischen Einsatzort, umfassend:
eine erste Filtereinheit, die zum Trennen von Blutzellen aus einer Vollblutprobe ausgebildet ist;
eine zweite Filtereinheit, die zum Trennen von Zellfragmenten, einschließlich Blutplättchen oder Exosomen, ausgebildet ist; und
eine dritte Filtereinheit, die zum Trennen von Proteinen ausgebildet ist,
wobei die Filter der ersten, zweiten und dritten Einheit unterschiedliche Porengrößen aufweisen und nach abnehmender Porengröße hintereinander angeordnet sind und jeweils mit einem Zwischenraum voneinander beabstandet sind,
wobei die kombinierten Filtereinheiten so ausgebildet sind, dass sie zur Diagnose der jeweiligen Blutbestandteile zerlegt werden können,
und wobei die Vollblutprobe durch Durchlaufen der Filtereinheiten getrennt wird,
**dadurch gekennzeichnet, dass** die Porengröße der ersten Filtereinheit im Bereich von 5 µm bis 15 µm liegt, die Porengröße der zweiten Filtereinheit im Bereich von 0,05 µm bis 1 µm liegt und die Porengröße der dritten Filtereinheit im Bereich von 0,003 µm bis 0,01 µm liegt.

2. Der mehrschichtige Filter gemäß Anspruch 1,
weiter umfassend eine vierte Filtereinheit, die zum Trennen von kleinen Protein- oder Nukleinsäurebestandteilen ausgebildet ist, welche die dritte Filtereinheit durchlaufen haben,
wobei die vierte Filtereinheit vorzugsweise aus einem Siliciumdioxid-Material hergestellt ist, um eine verbesserte Adsorption von Nukleinsäuren zu ermöglichen.

3. Der mehrschichtige Filter gemäß einem der Ansprüche 1 oder 2,
wobei der mehrschichtige Filter ausgebildet ist, um eine in einer Menge von 20 bis 100 µL zugeführte Vollblutprobe zu trennen.

4. Der mehrschichtige Filter gemäß einem der Ansprüche 1 bis 3,
wobei die erste Filtereinheit eine Dicke von 500 bis 1500 µm, ein Gewicht von 100 bis 400 g/m², eine Filterfeinheit von 2 bis 5 µm und ein Rückhaltevolumen von 20 bis 100 µL aufweist.

5. Der mehrschichtige Filter gemäß Anspruch 4,
wobei die Porengröße 10 µm, die Dicke 1000 µm, das Gewicht 250 g/m², die Filterfeinheit 3 µm und das Rückhaltevolumen 50 µL beträgt.

6. Der mehrschichtige Filter gemäß einem der Ansprüche 1 bis 5,
wobei die zweite Filtereinheit eine Dicke von 100 bis 1000 µm, ein Gewicht von 100 bis 300 g/m², eine Filterfeinheit von 1 bis 5 µm und ein Rückhaltevolumen von 2 bis 10 µL aufweist.

7. Der mehrschichtige Filter gemäß Anspruch 6,
wobei die Porengröße 0,1 µm, die Dicke 300 µm, das Gewicht 200 g/m², die Filterfeinheit 2 µm und das Rückhaltevolumen 5 µL beträgt.

## Revendications

1. Filtre multicouche de séparation de composants sanguins immédiatement au niveau d'un centre médical, comprenant :
une première unité de filtre configurée pour séparer des cellules sanguines d'un échantillon de sang total ;
une deuxième unité de filtre configurée pour séparer des fragments cellulaires comprenant une plaquette sanguine ou un exosome ; et une troisième unité de filtre configurée pour séparer une protéine,
et dans lequel les filtres des première, deuxième et troisième unités ont des tailles de pores différentes et sont combinés successivement par taille de pores décroissante et sont espacés les uns des autres par un espace entre eux
et dans lequel les unités de filtre combinées sont configurées pour être désassemblées à des fins de diagnostic de chaque composant sanguin, et dans lequel l'échantillon de sang total est séparé par un passage à travers les unités de filtre,
**caractérisé en ce que** la taille de pores de la première unité de filtre s'inscrit dans une plage de 5 µm à 15 µm, et la taille de pores de la deuxième unité de filtre s'inscrit dans une plage de 0,05 µm à 1 µm, et la taille de pores de la troisième unité de filtre s'inscrit dans une plage de 0,003 µm à 0,01 µm.

2. Filtre multicouche selon la revendication 1, comprenant en outre :
une quatrième unité de filtre configurée pour séparer des composants de petite protéine ou d'acide nucléique ayant traversé la troisième unité de filtre et dans lequel la quatrième unité de filtre est, de préférence, constituée d'un matériau de silice à des fins d'adsorption améliorée d'acides nucléiques.

3. Filtre multicouche selon l'une ou l'autre des revendications 1 et 2, dans lequel le filtre multicouche est configuré pour séparer l'échantillon de sang total administré en une quantité de 20 à 100 µL.

4. Filtre multicouche selon l'une quelconque des revendications 1 à 3, dans lequel la première unité de filtre a une épaisseur de 500 à 1500 µm, un poids de 100 à 400 g/m², un degré de filtration de 2 à 5 µm, et un volume de rétention de 20 à 100 µL.

5. Filtre multicouche selon la revendication 4, dans lequel la taille de pores est de 10 µm, l'épaisseur est de 1000 µm, le poids est de 250 g/m², le degré de filtration est de 3 µm, et le volume de rétention est de 50 µL.

6. Filtre multicouche selon l'une quelconque des revendications 1 à 5, dans lequel la deuxième unité de filtre a une épaisseur de 100 à 1000 µm, un poids de 100 à 300 g/m², un degré de filtration de 1 à 5 µm, et un volume de rétention de 2 à 10 µL.

7. Filtre multicouche selon la revendication 6, dans lequel la taille de pores est de 0,1 µm, l'épaisseur est de 300 µm, le poids est de 200 g/m², le degré de filtration est de 2 µm, et le volume de rétention est de 5 µL.
